# EUROPEAN PATENT APPLICATION

(11) **EP 4 094 733 A1**
(43) Date of publication of application: **30.11.2022**
(21) Application number: 21176430.3
(22) Date of filing: 28.05.2021
(51) Int. Cl.: A61F 5/445, B26F 1/32, B26D 7/26

(54) **OSTOMY CARE SYSTEM AND METHOD FOR PREPARING A THROUGH OPENING IN A BASE PLATE OF AN OSTOMY CARE SYSTEM**

(71) Applicant: Aesculap AG, 78532 Tuttlingen (DE)
(72) Inventor: Ellenberger, Thomas, 78549 Spaichingen (DE); Schmid, Gerhard, 78194 Immendingen-Hattingen (DE)
(74) Representative: Hoeger, Stellrecht & Partner Patentanwälte mbB

(57) **Abstract**

The invention relates to an ostomy care system comprising a medical cutting device for cutting a through opening in a base plate of the ostomy care system, wherein the base plate is connected or connectable with a pouch, wherein the medical cutting device comprises a medical punch for blanking out or punching out the through opening in the base plate.

Further, the invention relates to a method for preparing a through opening in a base plate of an ostomy care system, wherein the base plate is connected or connectable with a pouch, wherein the through opening in the base plate is formed by blanking out or punching out.

## Description

The present invention relates to an ostomy care system comprising a medical cutting device for cutting a through opening in a base plate of the ostomy care system, wherein the base plate is connected or connectable with a pouch.

Further, the present invention relates to a method for preparing a through opening in a base plate of an ostomy care system, wherein the base plate is connected or connectable with a pouch.

Ostomy care systems, also referred to as ostomy pouching systems, provide a means for collecting waste from a surgically diverted biological system, for example, a colon, ileum or a bladder, in case of an artificial stoma. Pouching systems are most commonly associated with ostomies, ileostomies and urostomies.

Such pouching systems usually consist of a collection pouch, a barrier on the skin, in particular in the form of a base plate, and connect with the stoma itself, which is the part of the body that has been diverted to the skin. The system may be a one-piece system consisting only of a bag or, as described above, involve a device placed on the skin, namely the base plate, with a collection pouch that is attached mechanically or with an adhesive in an airtight seal. The latter is known as a two-piece system.

The base plate sits on the skin and separates the ostomy pouch from the internal conduit. The internal opening, also called the through opening, of the base plate must have a correct size to accommodate the individual's stoma while protecting the skin from contact with waste. This is important in order to avoid any contamination due to contact of the aggressive waste with the skin. Cutting out the base plate for preparing the through opening with a pair of scissors is time-consuming and demands certain skill of the user. However, any sharp edge remaining in the base plate delimiting the through opening can cause harm to the stoma which should be avoided.

Therefore, it is an object of the present invention to improve an ostomy care system and a method described in the outset in order to facilitate the preparation of the through opening in the base plate.

This object is achieved in accordance with the present invention in that the cutting device comprises a medical punch for blanking out or punching out the through opening in the base plate.

Providing a medical punch allows for the simple preparation of a through opening in a base plate. Punching the through opening does not demand great skill from a user. Further, such a punch can ensure that the rim of the base plate delimiting the through opening does not comprise any sharp projections which can irritate or cause harm to the stoma. In other words: the improved ostomy care system allows for preparing base plates which perfectly fit to an individual's stoma. The base plate can be easily and quickly prepared with the required through opening corresponding to the stoma.

Further, it is advantageous if the medical punch comprises a punch member, if the punch member comprises at least one handle and at least one cutting element with a cutting edge, if the handle forms a proximal part of the punch member, if the at least one cutting element forms a distal part of the punch member, and if the cutting edge defines a distal end of the punch member. Such a medical punch can be made as one piece, in particular monolithically. The handle allows for the handling of the medical punch by hand. The cutting element can be moved with the cutting edge towards the base plate to punch out the through opening. Alternatively, the at least one handle and the at least one cutting element can be formed separately and connected to one another by substance-to-substance bond or in a force-locking or positive-locking manner. Further, the ostomy care system can comprise two or more medical punches which differ, in particular in size and shape of the cutting edge. This allows for preparing through openings in base plates having different sizes and shapes. A further option is that the ostomy care system comprises a single handle and a plurality of cutting elements which can be selectively connected to the handle.

Through openings of high quality can be easily prepared if the cutting edge is designed in the form of a self-contained ring-shaped cutting edge.

Preferably, the cutting edge defines a circular or oval shape. This allows for preparing through openings having a circular or oval shape.

It is favorable if the least one handle and the at least one cutting element are releasably connectable to one another. This enables, in particular selective coupling of the at least one handle and the at least one cutting element with one another. In particular one or two or more handles can be selectively coupled to a cutting element chosen from a plurality of cutting elements which differ in size and/or shape of the cutting edge. Such an ostomy care system can be provided with a limited number of elements. In particular it can comprise only a single handle which helps to reduce the overall size of the ostomy care system. This is an advantage, in particular if the individual travels and has to carry the ostomy care system with it.

In order to selectively couple the at least one handle and the at least one cutting element, it is advantageous if the at least one punch member comprises a connecting device for releasably connecting the at least one handle and the at least one cutting element.

The ostomy care system can be easily manufactured if the connecting device is designed in the form a screw coupling or a bayonet connection. Thus, the at least one handle and the at least one cutting element can be easily coupled to one another.

Preferably, the connecting device comprises first and second connecting elements, wherein the first connecting element is arranged or formed on the handle portion, wherein the second connection element is arranged or formed on the at least one cutting element, and wherein the first and second connecting elements are in engagement in a connected position and are disengaged in a disconnected position. By bringing the first and second connective elements into engagement, the at least one handle and the at least one cutting element can be easily connected to one another in the connected position. In particular for cleaning purposes and for compact storage of the ostomy care system, the first and second connecting elements can be disengaged to clean or store them in the disconnected position.

For a simple and easy manufacturing of the connection device it is advantageous if one of the first and second connecting elements comprises an external thread and if the other of the first and second connecting elements comprises an internal thread corresponding to the external thread. This allows a user to simply screw the at least one handle and the at least one cutting element together.

Preferably, one of the first and second connecting elements comprises a first blind hole and the other of the first and second connecting elements comprises a projection corresponding to the first blind hole. This enables, in particular connecting the first and second connecting elements to one another in a force- and/or positive-locking manner. In particular, the first and second connecting elements can extend in a direction parallel or coaxially with a longitudinal axis of the punch member.

It is advantageous if the internal thread is formed in an inner wall of the first blind hole, and if the external thread is formed on an outer surface of the projection. Such a connecting device can be easily manufactured and handled by a user.

In a further embodiment, each of the at least two cutting elements has an identically formed second connecting element for connecting to the first connecting element of the handle portion. Such a construction allows for providing, in particular, only a single handle with a plurality of different cutting elements. Thus, the number of components of the ostomy care system can be significantly reduced. In particular, the weight and also the required space for storing the ostomy care system can be minimized.

Through openings in base plates can be easily prepared if the at least one cutting element is designed in the form of a cutting sleeve comprising a second blind hole extending from the distal end in a proximal direction. In other words, the at least one cutting element having such a design has a second blind hole which is open in distal direction. The second blind hole can be used to receive the piece in the form of a disk which has been cut out from the base plate.

Preferably, the second blind hole defines an inner cross-section which corresponds or substantially corresponds in form and size to a cross-section defined by the cutting edge. This allows for receiving the cut-out piece from the base plate in a simple manner.

It is favorable if the medical cutting device comprises at least two cutting elements whose cutting edges differ in size. This allows a user to make use of the respective cutting element which corresponds best to the size of the stoma. Thus, the base plate can be arranged perfectly fitting around the stoma on the individual's skin.

Further it is advantageous if the at least two cutting elements are of similar or different shape. For example, they can be of circular shape but with different cross-sections or defining different diameters. In this case they are similar in shape. However, they can also differ in shape so that one of the cutting elements has a circular shape and the other cutting element has an oval shape. The area defined by the respective cutting edges of the at least two cutting elements can be identical if the cutting edges have different shapes or they can differ in size if the cross-section of the cutting edges differs in size.

In order to allow an individual to fit the base plate best to its stoma, it is preferred if the medical cutting device comprises two, three, four, five, six or more cutting elements. With such different cutting elements, the through opening in the base plate can be provided in different sizes and/or shapes. Thus, an individual can prepare a base plate with a through opening that corresponds in size and/or shape with its stoma in the morning, and a second base plate with a through opening of a different size and/or shape which can fit to the stoma at another time of the day, in particular after a meal or after exercising.

In order to easily sharpen the cutting edge of the cutting element, it is preferred if an outer surface of the at least one cutting element tapers in the direction toward the cutting edge.

In accordance with a further embodiment, the medical cutting device defines a longitudinal axis and comprises a guide arrangement for guiding a movement of the punch member in a direction parallel to the longitudinal axis. The guide arrangement supports a user in moving the punch member in a defined manner towards the base plate for cutting out the through opening. Thus, the through opening can be easily prepared in the base plate in exactly the required position.

Preferably, the guide arrangement comprises a guide member movably arranged on the punch member and the guide member has a distal end for centering the medical punch with respect to the base plate. Such a guide member allows for being positioned on the base plate and for guiding the punch member with respect to the guide member when moving the cutting element with the cutting edge towards the base plate for cutting out or punching out the through opening.

The punch member can be perfectly guided if the distal end of the guide member has an outer contour corresponding to an outer contour of the cutting edge. This allows a user to position the guide member on the base plate such that the through opening will be exactly prepared around the outer contour of the guide member when the punch member is moved with the cutting edge towards the base plate.

Further, it may be advantageous if the guide member is movably arranged so as to protrude beyond the cutting edge in a distal direction in a centering position and so as to be retracted behind the cutting edge in proximal direction within the second blind hole in a punching position. Such a construction makes it possible to move the guide member into the centering position to bring it into contact with the base plate for centering the medical cutting device as desired. Then, the punch member can be moved in distal direction and beyond the guide member for cutting the base plate for preparing the desired through opening.

Preferably, the medical cutting comprises a biasing element for automatically moving the guide member into the punching position. For example, a user can hold back the punch member with respect to the guide member, position the guide member on the base plate and then release the punch member which will then be automatically moved towards the base plate for blanking out the through opening. Optionally, the medical cutting device can comprise a locking mechanism for locking the medical cutting device in the centering position. Preferably, the locking device can be released by an actuating member for automatically moving the punch member in distal direction upon activating the actuating member.

In accordance with a further preferred embodiment, the guide member is arranged so as to be movable from the punching position against the effect of the biasing element into the centering position. This means, in particular that moving the guide member into the punching position automatically pretensions the biasing element. Such a pretensioning can be used for automatically moving the punch member relative to the guide member in distal direction for punching out the through opening in the base plate.

The ostomy care system can be simply manufactured if the biasing element is designed in the form of a spring. In particular, the spring can be in the form of a coil spring. Further, the spring can be designed in the form of a compression spring or in the form a tension spring depending on the construction of the medical cutting device.

Preferably, the biasing element in the centering position takes a more tensioned state than in the punching position. Such a design allows for a simple construction of the medical cutting device, which, in particular allows for automatically moving the punch member in distal direction from the centering position to the punching position.

It is advantageous, if the punch member is provided with a longitudinal guide channel extending coaxially with the longitudinal axis from a proximal end of the punch member to a distal end thereof, if the guide arrangement comprises a guide shaft slidably arranged in the guide channel, and if the guide member is arranged on a distal end of the guide shaft. Such a design enables a simple handling of the medical cutting device. In particular, the punch member can be easily slid on the guide shaft in distal and proximal direction. Thus, the guide member can be positioned on the base plate and the punch member can be moved relative both to the guide shaft and the guide member in distal direction in a defined manner.

A simple design of the medical cutting device can be made possible if the biasing element is arranged so as to surround the guide shaft. In other words, the guide shaft extends through the biasing element along its longitudinal axis.

Preferably, the guide channel defines a fluid communication between the first blind hole and the second blind hole of the at least one cutting element. This allows for the at least one cutting element to be guided on the guide shaft and to be moved in proximal and distal direction as desired with respect to the guide shaft.

In accordance with a further preferred embodiment, the guide arrangement comprises a stop member for limiting a movement of the guide member in distal direction. This construction, in particular enables the guide member to be moved beyond the cutting edge only in a limited way, i.e. into a most distal position when the stop member, for example, abuts the punch member.

A simple handling of the medical cutting device is possible if the stop member is arranged or formed on a proximal end of the guide shaft. This allows an individual to use the stop member, in particular as a handle portion for moving the guide shaft connected to the stop member with respect to the punch member. It should be noted that the stop member and the guide shaft can also be made from one piece, in particular monolithically.

The ostomy care system can be easily cleaned it the stop member is releasably connectable to the guide shaft. Thus, the guide shaft and the stop member can be disengaged, and, in particular the guide shaft can optionally be disengaged from the punch member.

The guide shaft and the stop member can be easily disengaged, if the guide arrangement comprises a screwing connection for connecting the stop member and the guide shaft.

A simple construction of the medical cutting device can be made possible if the biasing element is arranged between a proximal end of the punch member and the stop member. If the biasing element is designed in the form of a compression spring, the stop member can be moved towards the proximal end of the punch member against the effect of the biasing element. Releasing the stop member results in the biasing element moving the stop member in proximal direction away from the proximal end of the punch member, i.e. back into the punching position.

In accordance with a preferred embodiment, the ostomy care system comprises at least one base plate being connected or connectable with a pouch. The base plate is in an unblanked state, i.e. a user must blank out the through opening in a base plate before it can be used with a stoma. As already described above, the user has the option to prepare the through opening in size and/or shape so that it fits best with the stoma.

Preferably, the at least one base plate is provided with at least one marking indicating a form and size which corresponds to the at least one cutting element of the punch member. In particular, the base plate can be provided with two or more markings corresponding to different cutting elements of the medical cutting device. Thus, the handling of the medical cutting device can be facilitated and the through opening can be easily controlled with respect to size and/or shape.

Further, it is preferred if the ostomy care system comprises at least one pouch connectable with the base plate. A user can connect the pouch with the base plate for collecting waste leaving the stoma.

The object described in the outset can be further achieved in a method set forth in the outset in that the through opening in the base plate is formed by blanking out or punching out.

As already described above, preparing the through opening in the base plate in the proposed manner is simple and results in a perfect rim of the through opening without any protruding sharp edges.

Preferably, size and/or shape of the stoma are determined, and the through opening is prepared in size and/or shape so as to fit to the stoma. This improved method can be used, in particular in connection with an ostomy care system described above. In particular, after determining size and/or shape of the stoma, the best-suited cutting element as far as size and/or shape is concerned of the ostomy care system can be chosen for punching out the through opening in the base plate.

Further, a use of an ostomy care system described above for carrying out a method for preparing a through opening in a base plate as described above is proposed.

This allows for simply preparing a base plate which fits best to an individual's stoma.

Hence, the foregoing description comprises, in particular the embodiments of ostomy care systems and methods for preparing a through opening in a base plate of an ostomy care system, which are defined hereinafter in the form of numbered sentences:
1. Ostomy care system (28) comprising a medical cutting device (30) for cutting a through opening (20) in a base plate (18) of the ostomy care system (28), wherein the base plate (18) is connected or connectable with a pouch (24), characterized in that the medical cutting device (30) comprises a medical punch (32) for blanking out or punching out the through opening (20) in the base plate (18).
2. Ostomy care system according to sentence 1, characterized in that the medical punch (32) comprises a punch member (34), in that the punch member (34) comprises at least one handle (36) and at least one cutting element (38, 38a, 38b, 38c) with a cutting edge (42), in that the handle (36) forms a proximal part of the punch member (34), in that the at least one cutting element (38, 38a, 38b, 38c) forms a distal part of the punch member (34), and in that the cutting edge (42) defines a distal end (40) of the punch member (34).
3. Ostomy care system according to sentence 2, characterized in that the cutting edge (42) is designed in the form of a self-contained ring-shaped cutting edge (42).
4. Ostomy care system according to sentence 2 or 3, characterized in that the cutting edge (42) defines a circular or oval shape.
5. Ostomy care system according to any one of sentences 2 to 4, characterized in that the at least one handle (36) and the at least one cutting element (38, 38a, 38b, 38c) are releasably connectable to one another.
6. Ostomy care system according to any one of sentences 2 to 4, characterized in that the at least one punch member (34) comprises a connecting device (64) for releasably connecting the at least one handle (36) and the at least one cutting element (38, 38a, 38b, 38c).
7. Ostomy care system according to sentence 6, characterized in that the connecting device (64) is designed in the form of a screw coupling (66) or a bayonet connection.
8. Ostomy care system according to sentence 6 or 7, characterized in that the connecting device (64) comprises first and second connecting elements (68, 70), in that the first connecting element (68) is arranged or formed on the handle (36), in that the second connecting element (70) is arranged or formed on the at least one cutting element (38, 38a, 38b, 38c), and in that the first and second connecting elements (68, 70) are in engagement in a connected position and are disengaged in a disconnected position.
9. Ostomy care system according to sentence 8, characterized in that one of the first and second connecting elements (68, 70) comprises an external thread (76) and in that the other of the first and second connecting elements (68, 70) comprises an internal thread (78) corresponding to the external thread (76).
10. Ostomy care system according to sentence 8 or 9, characterized in that one of the first and second connecting elements (68, 70) comprises a first blind hole (46) and in that the other of the first and second connecting elements (68, 70) comprises a projection (72) corresponding to the first blind hole (46).
11. Ostomy care system according to sentence 10, characterized in that the internal thread (78) is formed in an inner wall (80) of the first blind hole (46), and in that the external thread (76) is formed on an outer surface of the projection (72).
12. Ostomy care system according to any one of sentences 8 to 11, characterized in that each of the at least two cutting elements (38, 38a, 38b, 38c) has an identically formed second connecting element (70) for connecting to the first connecting element (68) of the handle (36).
13. Ostomy care system according to any one of sentences 2 to 12, characterized in that the at least one cutting element (38, 38a, 38b, 38c) is designed in the form of a cutting sleeve (44) comprising a second blind hole (48) extending from the distal end (40) in a proximal direction.
14. Ostomy care system according to sentence 13, characterized in that the second blind hole (48) defines an inner cross-section (60) which corresponds or substantially corresponds in form and size to a cross-section (62) defined by the cutting edge (42).
15. Ostomy care system according to any one of sentences 2 to 14, characterized in that the medical cutting device (30) comprises at least two cutting elements (38, 38a, 38b, 38c) whose cutting edges (42) differ in size.
16. Ostomy care system according to sentence 15, characterized in that the at least two cutting elements (38, 38a, 38b, 38c) are of similar or different shape.
17. Ostomy care system according to any one of sentences 2 to 16, characterized in that the medical cutting device (30) comprises two, three, four, five, six or more cutting elements (38, 38a, 38b, 38c).
18. Ostomy care system according to any one of sentences 2 to 17, characterized in that an outer surface (58) of the at least one cutting element (38, 38a, 38b, 38c) tapers in the direction toward the cutting edge (42).
19. Ostomy care system according to any one of sentences 2 to 18, characterized in that the medical cutting device (30) defines a longitudinal axis (52) and comprises a guide arrangement (82) for guiding a movement of the punch member (34) in a direction parallel to the longitudinal axis (52).
20. Ostomy care system according to sentence 19, characterized in that the guide arrangement (82) comprises a guide member (84) movably arranged on the punch member (34) and in that the guide member (84) has a distal end (86) for centering the medical punch (32) with respect to the base plate (18).
21. Ostomy care system according to sentence 20, characterized in that the distal end (86) of the guide member (84) has an outer contour corresponding to an outer contour of the cutting edge (42).
22. Ostomy care system according to sentence 20 or 21, characterized in that the guide member (84) is movably arranged so as to protrude beyond the cutting edge (42) in a distal direction in a centering position and so as to be retracted behind the cutting edge (42) in proximal direction within the second blind hole (48) in a punching position.
23. Ostomy care system according to sentence 22, characterized in that medical cutting device (30) comprises a biasing element (114) for automatically moving the guide member (84) in the punching position.
24. Ostomy care system according to sentence 23, characterized in that the guide member (84) is arranged so as to be movable from the punching position against the effect of the biasing element (114) into the centering position.
25. Ostomy care system according to 23 or 24, characterized in that the biasing element (114) is designed in the form of a spring (116), in particular in the form of a coil spring (118).
26. Ostomy care system according to any one of sentences 23 to 25, characterized in that the biasing element (114) in the centering position takes a more tensioned state than in the punching position.
27. Ostomy care system according to any one of sentences 20 to 26, characterized in that the punch member (34) is provided with a longitudinal guide channel (90) extending coaxially with the longitudinal axis (52) from a proximal end (92) of the punch member (34) to a distal end (40) thereof, in that the guide arrangement (82) comprises a guide shaft (88) slidably arranged in the guide channel (90), and in that the guide member (84) is arranged on a distal end (94) of the guide shaft (88).
28. Ostomy care system according to sentence 27, characterized in that the biasing element (114) is arranged so as to surround the guide shaft (88).
29. Ostomy care system according to sentence 27 or 28, characterized in that the guide channel (90) defines a fluid communication between the first blind hole (46) and the second blind hole (48) of the at least one cutting element (38, 38a, 38b, 38c).
30. Ostomy care system according to any one of sentences 20 to 29, characterized in that the guide arrangement (82) comprises a stop member (110) for limiting a movement of the guide member (84) in distal direction.
31. Ostomy care system according to sentence 30, characterized in that the stop member (110) is arranged or formed on a proximal end (102) of the guide shaft (88).
32. Ostomy care system according to sentence 30 or 31, characterized in that the stop member (110) is releasably connectable to the guide shaft (88).
33. Ostomy care system according to any one of sentences 30 to 32, characterized in that the guide arrangement (82) comprises a screwing connection (120) for connecting the stop member (110) and the guide shaft (88).
34. Ostomy care system according to any one of sentences 30 to 33, characterized in that the biasing element (114) is arranged between a proximal end (92) of the punch member (34) and the stop member (110).
35. Ostomy care system according to any one of the preceding sentences, characterized in that it comprises at least one base plate (18) being connected or connectable with a pouch (24).
36. Ostomy care system according to sentence 35, characterized in that the at least base plate (18) is provided with at least one marking (124, 124a, 124b, 124c) indicating a form and size which corresponds to the at least one cutting element of the punch member.
37. Ostomy care system according to any one of the preceding sentences, characterized in that the ostomy care system (30) comprises at least one pouch (24) connectable with the base plate (18).
38. Method for preparing a through opening (20) in a base plate (18) of an ostomy care system (28), wherein the base plate (18) is connected or connectable with a pouch (24), characterized in that the through opening (20) in the base plate (18) is formed by blanking out or punching out.
39. Method according to sentence 38, characterized in that size and/or shape of the stoma (10) are determined, and in that the through opening (20) is prepared in size and/or shape so as to fit to the stoma (10).
40. Use of an ostomy care system (28) in accordance with any one of sentences 1 to 37 for carrying out a method according to sentence 38 or 39.

The foregoing summary and the following description may be better understood in conjunction with the drawing figures of which:
- Figure 1:: shows a schematic depiction of an individual and an example of a stoma being part of a colon diverted to the individual's skin;
- Figure 2:: shows a schematic longitudinal sectional view of an embodiment of a medical cutting device;
- Figure 3:: shows a schematic perspective exploded view of the medical cutting device shown in Figure 2;
- Figure 4:: shows a schematic perspective exploded view of the punch member shown in Figure 3 with a handle disconnected from a cutting element;
- Figure 5:: shows a schematic longitudinal sectional view of the punch member shown in Figure 4;
- Figure 6:: shows a schematic depiction of the medical cutting device set with a guide member thereof on a blank of a base plate in a centering position;
- Figure 7:: shows a schematic depiction of the arrangement shown in Figure 6 with the punch member in the punching position blanking out a through opening in the base plate;
- Figure 8:: shows a schematic partial sectional view of the arrangement in Figure 7;
- Figure 9:: shows a schematic depiction of the arrangement in Figure 8 after lifting the medical punch from the base plate;
- Figure 10:: shows schematic sectional views of four cutting elements different in size (a) to d)) and an anvil plate supporting an embodiment of a base plate with a punched out through opening, the base plate having additional markings corresponding to the cutting elements shown in b) to d); and

- Figure 11:: shows an ostomy care system received in a storage and transportation case.

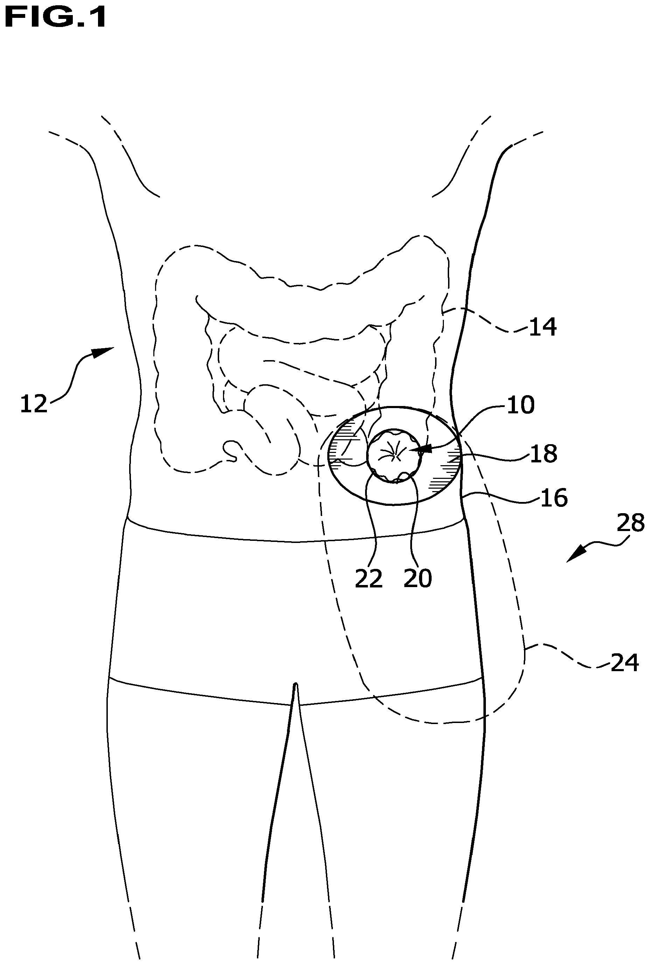

Figure 1 schematically depicts an artificial stoma as a result of a colostomy of a patient's 12 colon 14. In other words, the colon is diverted to the patient's 12 skin 16.

For collecting waste delivered from the colon 14, a base plate 18 provided with a through opening 20 surrounds the stoma 10. The free end 22 of the stoma passes through the through opening 20.

The waste is collected in a pouch 24 which is either made in one piece with the base plate 18 or provided separately and connected to the base plate 18 in a fluid tight manner.

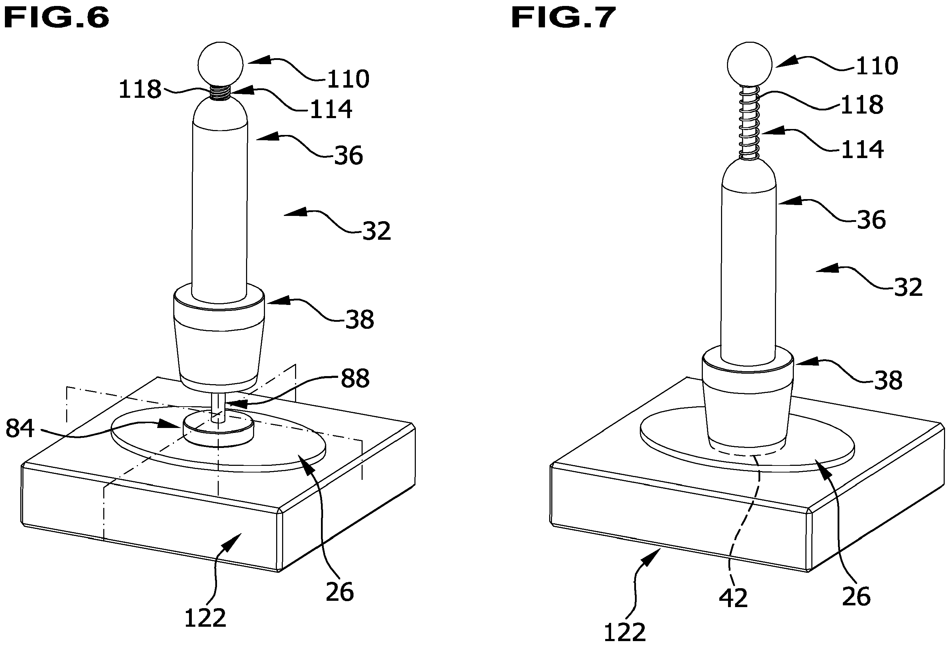

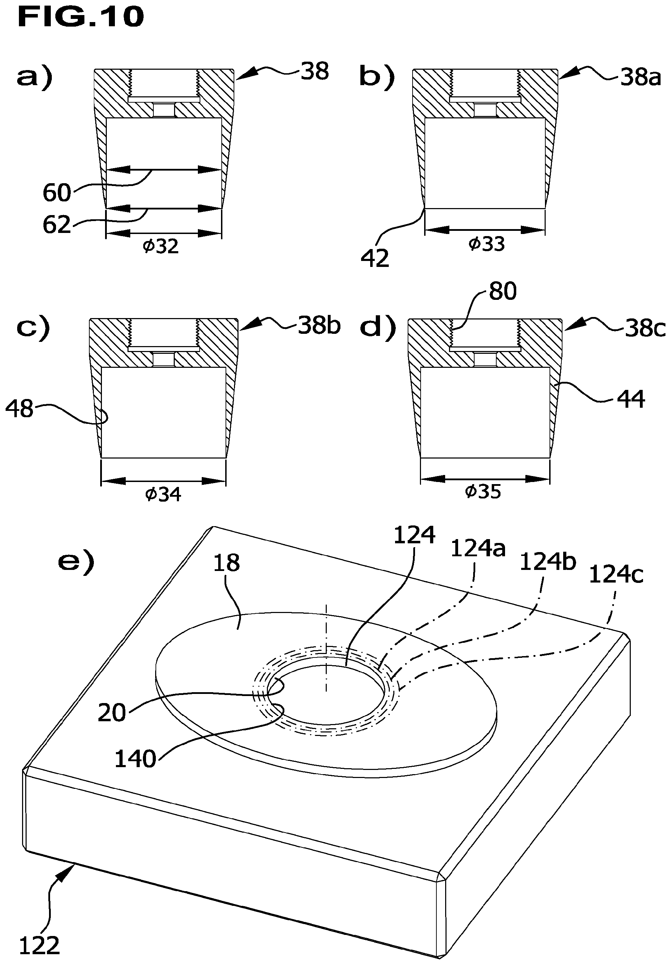

An embodiment of a base plate 18 for applying to the patient's 12 skin 16 is shown by way of example in Figure 10 e). The base plate 18 and, in particular the through opening 20, are prepared from a disc shaped blank 26 as shown, for example, in Figure 6.

An ostomy care system 28 comprises a medical cutting device 30 for cutting the through opening 20 in the base plate 18. The medical cutting device 30 comprises a medical punch 32 for blanking out or punching out the through opening 20 in the blank 26 for preparing the base plate 18 which can be applied to the stoma 10.

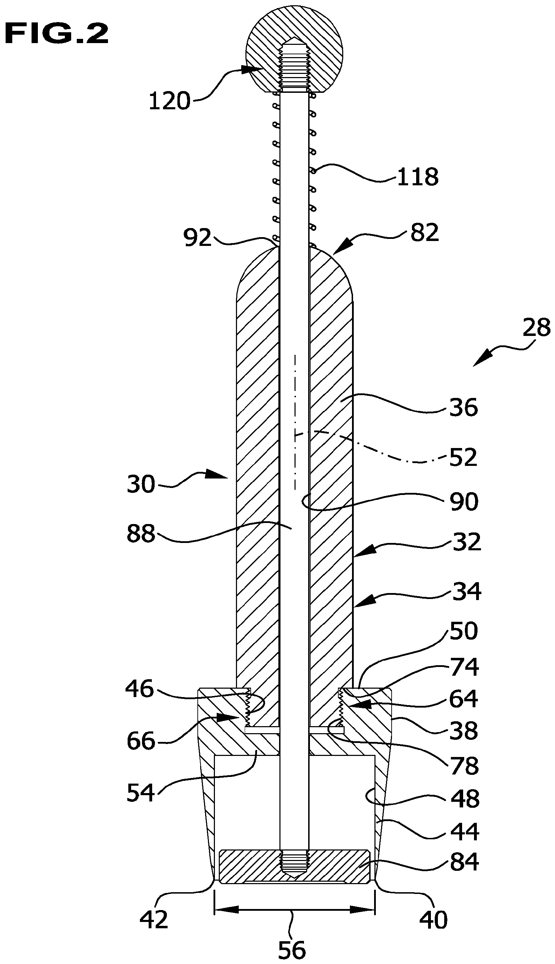

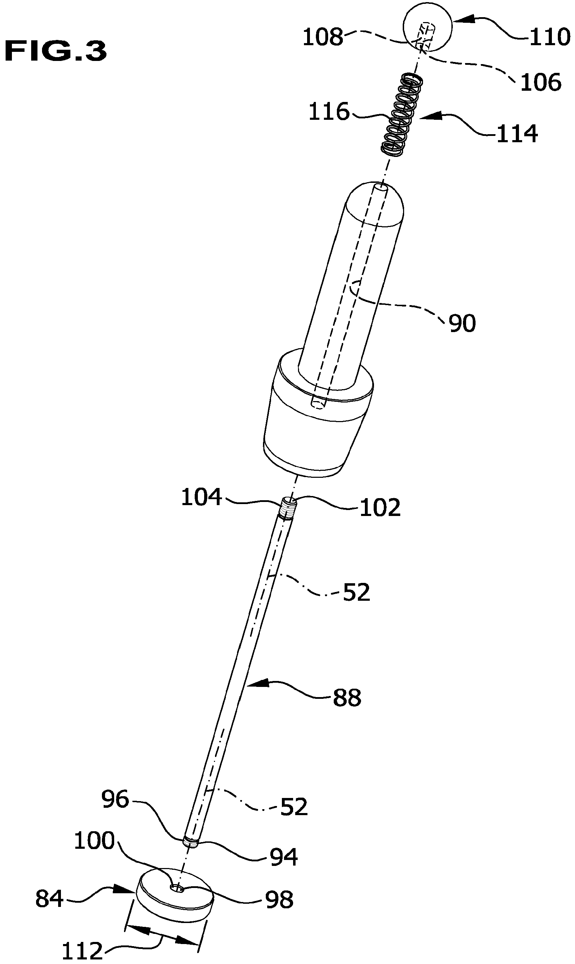

The construction and the function of the medical cutting device 30 will be further described in connection with Figures 2 to 11.

The medical punch 32 comprises a punch member 34 with a handle 36 and a cutting element 38. A distal end 40 of the cutting element 38 forms a cutting edge 42.

The handle 36 forms a proximal part or proximal portion of the punch member 34. The cutting element 38 forms a distal part or distal portion of the punch member 34.

The cutting edge 42 is formed self-contained and ring-shaped and has a circular or substantially circular shape.

The embodiments shown in the drawing figures comprise punch members 34 with the handle 36 and the cutting element 38 being releasably connectable to one another.

The cutting element 38 is designed in the form of a cutting sleeve 44 comprising a first blind hole 46 and a second blind hole 48. The first blind hole 46 is open in proximal direction and extends in distal direction starting from a proximal end surface 50 of the cutting element 38 in a direction parallel to a longitudinal axis 52 of the medical cutting device 30.

The second blind hole 48 is open in distal direction and extends in proximal direction from the cutting edge 42 in a direction parallel to the longitudinal axis 52. The first blind hole 46 and the second blind hole 48 are separated by a wall 54 extending in a direction transverse to the longitudinal axis 52.

A cross-section of the second blind hole 48 is defined by a diameter 56 of the cutting edge 42. Thus, the second blind hole 48 takes a hollow cylindrical form.

An outer surface 58 of the cutting element 38 tapers in the direction toward the cutting edge 42.

The embodiments shown in the drawing figures have second blind holes 48 defining an inner cross-section 60 which corresponds or substantially corresponds in form and size to a cross-section 62 defined by the cutting edge 42.

For releasably coupling the handle 36 and the cutting element 38, the punch member 34 comprises a connecting device 64. The connecting device 64 shown in connection with the embodiments of the drawing figures is designed in the form of a screw coupling 66. Alternative embodiments not shown in the drawing figures have connecting devices 64 in the form of bayonet connections.

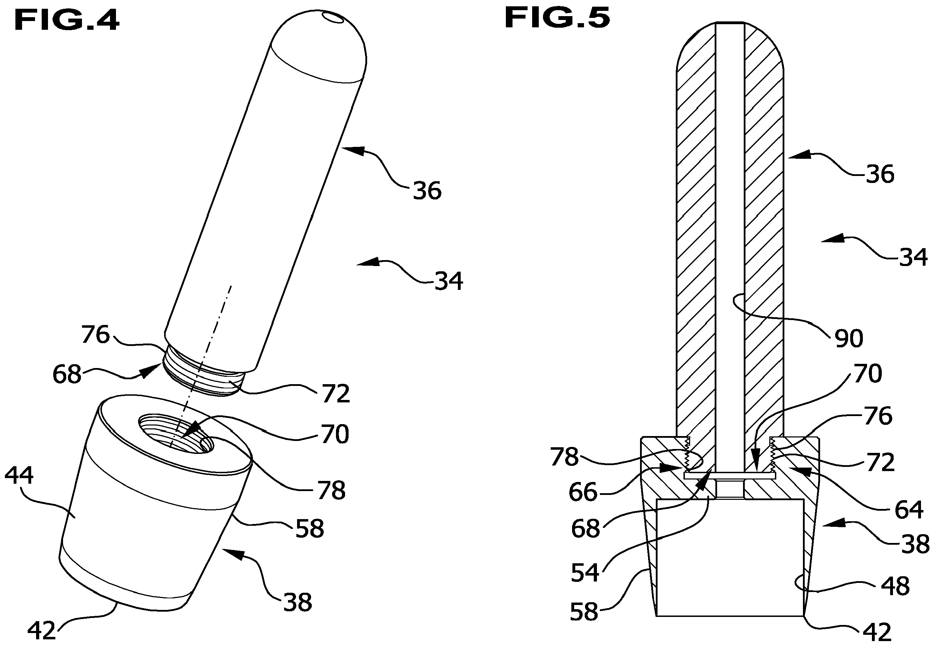

The connecting device 64 comprises first and second connecting elements 68, 70. The first connecting element 68 is arranged or formed on the handle 36. The second connecting element 70 is arranged or formed on the cutting element 38. The first and second connecting elements 68, 70 are in engagement in connected position as shown, for example, in Figure 5. They are disengaged in a disconnected position as shown, for example, in Figure 4.

The first and second connecting elements 68, 70 are engaged in a force-locking and/or positive-locking manner in the connected position. Alternative embodiments provide a substance-to-substance bond between the first and second connecting elements 68, 70 in order to maintain a permanent connection between the handle 36 and the cutting element 38.

The embodiments of the punch members 34 shown in the drawing figures have first connecting elements 68 in the form of projections 72 extending coaxially with the longitudinal axis 52 and pointing in distal direction. The projection 72 corresponds in form and shape with the first blind hole 68 in order to engage with this in a force-locking and/or positive-locking manner.

The second connecting element comprises the first blind hole 46. As the projection 72 extends beyond a distal end face 74 abutting the proximal end face 50 of the cutting element 38 in the connected position, the projection 72 extends in distal direction coaxially with the longitudinal axis 52.

The projection 72 is provided with an external thread 76. The first blind hole 46 comprises an internal thread 78 corresponding to the external thread 76. This allows for screwing the projection 72 into the first blind hole 46. The internal thread 78 is formed in an inner wall 80 of the first blind hole 46. The inner wall coaxially surrounds the longitudinal axis 52.

The medical cutting device 30 comprises a guide arrangement 82 for guiding a movement of the punch member 34 in a direction parallel to the longitudinal axis 52.

The guide arrangement 82 comprises a guide member 84 movably arranged on the punch member 34. A distal end 86 of the guide member 84 is provided for centering the medical punch 32 with respect to the base plate 18, namely the blank 26 in which the through opening 20 is to be prepared. The distal end 86 of the disc-shaped guide member 84 has an outer contour corresponding to or substantially corresponding to an outer contour of the cutting edge 42.

The guide arrangement 82 comprises a cylindrical guide shaft 88 which is slidably received in a guide channel 90. The guide channel 90 extends coaxially with the longitudinal axis 52 through the punch member 34 from a proximal end 92 of the punch member 34 to the distal end 40 thereof formed by the cutting edge 42. The guide channel 90 defines a fluid communication between the first blind hole 46 and the second blind hole 48 of the cutting element 38.

The guide member 84 is arranged on a distal end 94 of the guide shaft 88. For releasably connecting the guide member 84 to the guide shaft 88, the distal end 94 is provided with an external thread 96 corresponding to an internal thread 98 provided in a blind hole 100 of the guide member 84.

A proximal end 102 of the guide shaft 88 is provided with an external thread 104 corresponding to an internal thread 106 formed in a blind hole 108 of a stop member 110. Thus, stop member 110, which has the form of a sphere, can be screwed on the proximal end 102 of the guide shaft 88.

The guide member 84 forms a stop member for limiting a movement of the guide shaft 88 in proximal direction relative to the punch member 34. As an outer diameter 112 of the guide member 84 is smaller than the diameter 56, the guide member 84 can move in proximal direction until it abuts the wall 54. If this happens, the proximal end 102 of the guide shaft 88 protrudes beyond the proximal end 92 of the handle 36.

If the guide shaft 88 is moved in distal direction relative to the punch member 34, the stop member 110 abuts the proximal end 92 of the handle 36 and thus limits a movement in the distal direction. In this position, the guide member 84 protrudes beyond the cutting edge 42 as shown, for example, in Figure 6.

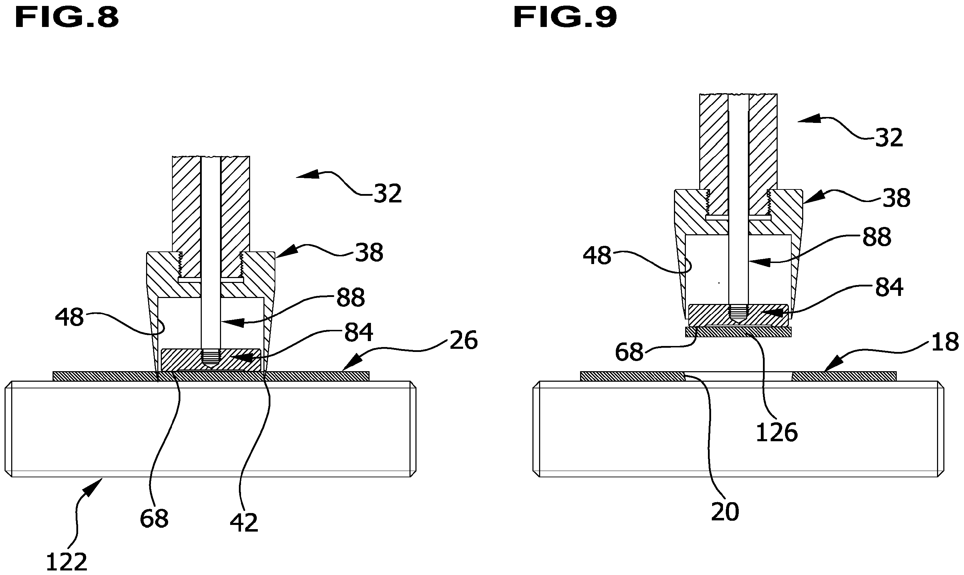

As described, the guide member 84 is movably arranged so as to protrude beyond the cutting edge 42 in a distal direction in a centering position as shown in Figure 6 and so as to be retracted behind the cutting edge 42 in a proximal direction within the second blind hole 48 in a punching position as shown in Figure 8.

The medical cutting device 30 comprises a biasing element 114 for automatically moving the guide member 84 into the punching position. The biasing element 114 is designed in the form of a spring 116. The embodiments shown in the drawing figures comprise a spring 116 in the form of a coil spring 118.

The biasing element 114 is arranged so as to surround the guide shaft 88 as shown, for example, in Figure 2. Further, the biasing element 114 is arranged between the proximal end 92 of the punch member 34 and a distal side of the stop member 110.

The described construction allows for the movement of the guide member 84 from the punching position against the effect of the biasing element 114 into the centering position. For this, a user holding the handle 36 with one hand can move the stop member 110 in distal direction towards the proximal end 92, thereby compressing the coil spring 118. The centering position with the compressed coil spring is shown, for example, in Figure 6.

As described above, the stop member 110 is releasably connectable to the guide shaft 88. The internal thread 106 and the external thread 104 corresponding to the internal thread 106 form a screwing connection for releasably connecting the stop member 110 and the guide shaft 88.

A use of the ostomy care system 28 for preparing a through opening 20 in a base plate 18 will be described in the following.

It is important to note that the size of the stoma 10 varies during a day. Due to manual work the diameter of the stoma 10 can be larger by several millimeters compared to the diameter of the stoma 10 in the morning. Further, temperature has an effect for the stoma's 10 size as well.

Therefore, the embodiment of the medical cutting device 30 can comprise more than one cutting element 34. The embodiment shown in the drawing figures comprises four cutting elements, namely cutting element 38 with a minimum cross-sectional diameter of the cutting edge 42 of 32 mm, and the cutting elements 38a, 38b and 38c shown in figure 10 with cross-sectional diameters of their cutting edges 42 of 33 mm, 34 mm and 35 mm.

All the four cutting elements 38, 38a, 38b and 38c are of similar construction, and, therefore, can be coupled with the handle 36 as set forth above. As outlined, the medical cutting device 30 comprises four cutting elements 38, 38a, 38b and 38c whose cutting edges 42 differ in size. However, the shape of the cutting edges 42 is similar, namely circular or substantially circular. Alternative embodiments of cutting elements 38 may have cutting edges 42 of different shapes, for example oval shapes.

Further, alternative embodiments can comprise more than or fewer than the four cutting elements 38, 38a, 38b and 38c.

For obtaining a base plate 18 which perfectly fits to the size of the stoma 10, the size of the stoma 10 is determined, for example with a jig or a measuring tape or the like. Then, depending on the size and the shape of the stoma 10, one of the cutting elements 38, 38a, 38b and 38c is chosen whose cross-sectional diameter of the cutting edge 42 corresponds best, i.e. is closest, to the determined size of the stoma 10.

The chosen cutting element 38 is connected to the handle 36. The guide member 84 is screwed on the distal end 94 of the guide shaft 88 and the proximal end 102 is introduced into the guide channel 90 through the wall 54.

The biasing element 114 is slid over the proximal end 102 and the stop member 110 is screwed thereon. As described above, medical punch 32 is now ready for preparing the through opening 20 in the base plate 10.

For this, the blank 26 is positioned on an anvil plate 122 as shown in Figure 6. The blank 26 can optionally be provided with marking rings 124, 124a, 124b and 124c as shown in Figure 10e) which correspond to the cross-sectional diameters of the respective cutting elements 38, 38a, 38b and 38c of the medical cutting device 30.

The guide member 84 is moved into the centering position and positioned on the blank 26 so as to be centered within the concentric marking rings 124, 124a, 124b and 124c. In the centering position, the punch member 34 takes its most proximal position with respect to the guide member 84. If the punch member 34 is released, the biasing member element 114 moves the punch member 34 in distal direction to thereby punch out or blank out the through opening 20 as shown in Figures 7 and 8.

The biasing element 114 automatically moves the guide member 84 in proximal direction so that it is received within the second blind hole 48 as shown in Figure 8.

After removal of the medical punch 32 from the anvil plate 122, the cut-out disc 126 is clampingly held at the cutting edge 42 within the second blind hole 48. Pushing the stop member 110 in distal direction ejects the disc 126 out of the second blind hole 48 as shown in Figure 9. Thus, the guide arrangement 82 serves the function of an ejector for ejecting the disc 126 out of the second blind hole 48.

The base plate 18 is now ready to be applied to the stoma 10. The pouch 24 can then be attached to the base plate 18 for receiving waste leaving the stoma 10.

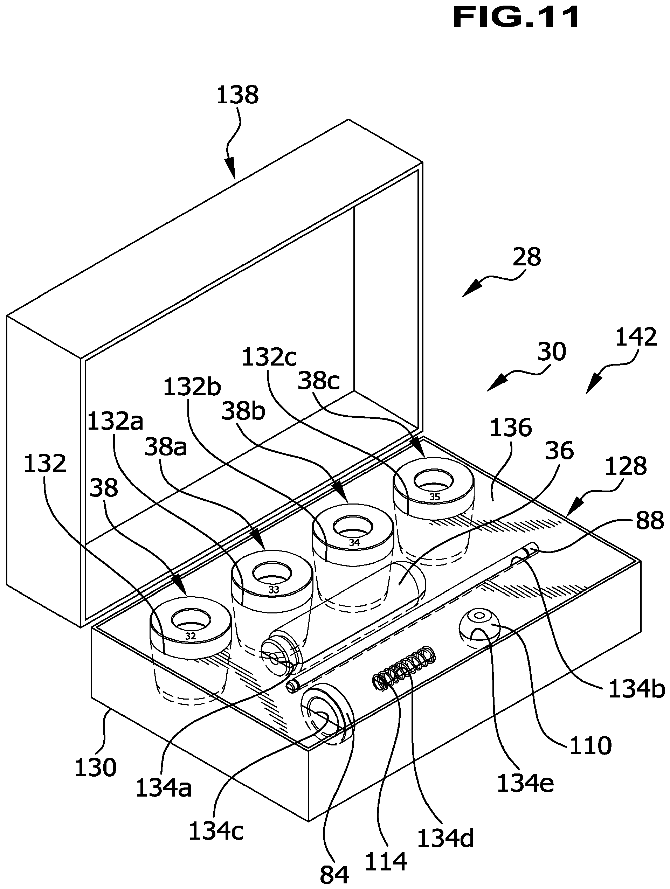

The medical cutting device 30 which forms a modular set 142 for providing medical punches 32 of different sizes, namely different sizes of the respective cutting edges 42, is shown in Figure 11 with its components received in a transportation and storage case 128. The case 128 comprises a container bottom 130 with receptacles 132, 132a, 132b and 132c for receiving the four cutting elements 38, 38a, 38b and 38c. Further receptacles 134, 134a, 134b, 134c, 134d and 134e are provided for receiving the handle 36, the guide shaft 88, the guide member 84, the biasing element 114 and the stop member 110. The receptacles 132 and 134 are formed in a board 136 made from a plastics. The board 136 is received in the container bottom 130. A lid 138 is provided for closing the container bottom 130.

As described and shown in the drawing figures, the medical cutting device 30 can be easily disassembled, in particular for cleaning and storing it.

The medical cutting device 30 allows for preparing through openings 20 of different sizes corresponding to the actual size of the stoma 10 in a simple manner. Compared to cutting out the through opening 20 with a pair of scissors, an inner rim 140 of the through opening 20 is perfectly shaped without any sharp edges and projections which could cause harm to the stoma 10.

All components of the medical cutting device 30 are made either of a sterilizable metal or sterilizable plastics. The cutting elements 38 are made from metal, the handle 36 is either made from a metal or plastics. The guide member 34 and the stop member 110 are either made from a metal or plastics. The guide shaft 88 and the biasing element are made from a metal.

### List of reference numerals

- 10: stoma
- 12: patient
- 14: colon
- 16: skin
- 18: base plate
- 20: through opening
- 22: end
- 24: pouch
- 26: blank
- 28: ostomy care system
- 30: medical cutting device
- 32: medical punch
- 34: punch member
- 36: handle
- 38, 38a, 38b, 38c: cutting element
- 40: distal end
- 42: cutting edge
- 44: cutting sleeve
- 46: first blind hole
- 48: second blind hole
- 50: proximal end surface
- 52: longitudinal axis
- 54: wall
- 56: diameter
- 58: surface
- 60: inner cross-section
- 62: cross-section
- 64: connecting device
- 66: screw coupling
- 68: first connecting element
- 70: second connecting element
- 72: projection
- 74: distal end face
- 76: external thread
- 78: internal thread
- 80: wall
- 82: guide arrangement
- 84: guide member
- 86: distal end
- 88: guide shaft
- 90: guide channel
- 92: proximal end
- 94: distal end
- 96: external thread
- 98: internal thread
- 100: blind hole
- 102: proximal end
- 104: external thread
- 106: internal thread
- 108: blind hole
- 110: stop member
- 112: diameter
- 114: biasing element
- 116: spring
- 118: coil spring
- 120: screwing connection
- 122: anvil plate
- 124, 124a, 124b, 124c: marking ring
- 126: disc
- 128: case
- 130: container bottom
- 132, 132a, 132b, 132c: receptacle
- 134a, 134b, 134c, 134d, 134e: receptacle
- 136: board
- 138: lid
- 140: rim
- 142: modular set

## Claims

1. Ostomy care system (28) comprising a medical cutting device (30) for cutting a through opening (20) in a base plate (18) of the ostomy care system (28), wherein the base plate (18) is connected or connectable with a pouch (24), **characterized in that** the medical cutting device (30) comprises a medical punch (32) for blanking out or punching out the through opening (20) in the base plate (18).

2. Ostomy care system according to claim 1, **characterized in that** the medical punch (32) comprises a punch member (34), **in that** the punch member (34) comprises at least one handle (36) and at least one cutting element (38, 38a, 38b, 38c) with a cutting edge (42), **in that** the handle (36) forms a proximal part of the punch member (34), **in that** the at least one cutting element (38, 38a, 38b, 38c) forms a distal part of the punch member (34), and **in that** the cutting edge (42) defines a distal end (40) of the punch member (34),
wherein, in particular the cutting edge (42)
a) is designed in the form of a self-contained ring-shaped cutting edge (42)
and/or
b) defines a circular or oval shape.

3. Ostomy care system according to claim 2, **characterized in that** the at least one handle (36) and the at least one cutting element (38, 38a, 38b, 38c) are releasably connectable to one another.

4. Ostomy care system according to claim 2 or 3, **characterized in that** the at least one punch member (34) comprises a connecting device (64) for releasably connecting the at least one handle (36) and the at least one cutting element (38, 38a, 38b, 38c),
wherein, in particular the connecting device (64)
a) is designed in the form of a screw coupling (66) or a bayonet connection
and/or
b) comprises first and second connecting elements (68, 70), **in that** the first connecting element (68) is arranged or formed on the handle (36), **in that** the second connecting element (70) is arranged or formed on the at least one cutting element (38, 38a, 38b, 38c), and **in that** the first and second connecting elements (68, 70) are in engagement in a connected position and are disengaged in a disconnected position,
wherein, in particular one of the first and second connecting elements (68, 70)
- comprises an external thread (76) and **in that** the other of the first and second connecting elements (68, 70) comprises an internal thread (78) corresponding to the external thread (76)
and/or
- comprises a first blind hole (46) and **in that** the other of the first and second connecting elements (68, 70) comprises a projection (72) corresponding to the first blind hole (46), wherein, in particular the internal thread (78) is formed in an inner wall (80) of the first blind hole (46), and **in that** the external thread (76) is formed on an outer surface of the projection (72).

5. Ostomy care system according to any one of claims 2 to 4, **characterized in that** at least one of
a) the medical cutting device (30) comprises at least two cutting elements (38, 38a, 38b, 38c) whose cutting edges (42) differ in size,
wherein the at least two cutting elements (38, 38a, 38b, 38c) are of similar or different shape;
b) the medical cutting device (30) comprises two, three, four, five, six or more cutting elements (38, 38a, 38b, 38c);
c) an outer surface (58) of the at least one cutting element (38, 38a, 38b, 38c) tapers in the direction toward the cutting edge (42).

6. Ostomy care system according to any one of claims 2 to 5, **characterized in that** the medical cutting device (30) defines a longitudinal axis (52) and comprises a guide arrangement (82) for guiding a movement of the punch member (34) in a direction parallel to the longitudinal axis (52).

7. Ostomy care system according to claim 6, **characterized in that** the guide arrangement (82) comprises a guide member (84) movably arranged on the punch member (34) and **in that** the guide member (84) has a distal end (86) for centering the medical punch (32) with respect to the base plate (18),
wherein, in particular the distal end (86) of the guide member (84) has an outer contour corresponding to an outer contour of the cutting edge (42).

8. Ostomy care system according to claim 7, **characterized in that** the guide member (84) is movably arranged so as to protrude beyond the cutting edge (42) in a distal direction in a centering position and so as to be retracted behind the cutting edge (42) in proximal direction within the second blind hole (48) in a punching position.

9. Ostomy care system according to claim 8, **characterized in that** medical cutting device (30) comprises a biasing element (114) for automatically moving the guide member (84) in the punching position,
wherein, in particular
a) the guide member (84) is arranged so as to be movable from the punching position against the effect of the biasing element (114) into the centering position
and/or
b) the biasing element (114) is designed in the form of a spring (116), in particular in the form of a coil spring (118)
and/or
c) the biasing element (114) in the centering position takes a more tensioned state than in the punching position.

10. Ostomy care system according to any one of claims 7 to 9, **characterized in that** the punch member (34) is provided with a longitudinal guide channel (90) extending coaxially with the longitudinal axis (52) from a proximal end (92) of the punch member (34) to a distal end (40) thereof, **in that** the guide arrangement (82) comprises a guide shaft (88) slidably arranged in the guide channel (90), and **in that** the guide member (84) is arranged on a distal end (94) of the guide shaft (88), wherein, in particular
a) the biasing element (114) is arranged so as to surround the guide shaft (88)
and/or
b) the guide channel (90) defines a fluid communication between the first blind hole (46) and the second blind hole (48) of the at least one cutting element (38, 38a, 38b, 38c).

11. Ostomy care system according to any one of claims 7 to 10, **characterized in that** the guide arrangement (82) comprises a stop member (110) for limiting a movement of the guide member (84) in distal direction,
wherein the stop member (110)
a) is arranged or formed on a proximal end (102) of the guide shaft (88)
and/or
b) is releasably connectable to the guide shaft (88).

12. Ostomy care system according to claim 11, **characterized in that**
a) the guide arrangement (82) comprises a screwing connection (120) for connecting the stop member (110) and the guide shaft (88)
and/or
b) the biasing element (114) is arranged between a proximal end (92) of the punch member (34) and the stop member (110).

13. Ostomy care system according to any one of the preceding claims, **characterized in that** the ostomy care system (30) comprises
a) at least one base plate (18) being connected or connectable with a pouch (24),
wherein, in particular the at least base plate (18) is provided with at least one marking (124, 124a, 124b, 124c) indicating a form and size which corresponds to the at least one cutting element of the punch member,
and/or
b) at least one pouch (24) connectable with the base plate (18).

14. Method for preparing a through opening (20) in a base plate (18) of an ostomy care system (28), wherein the base plate (18) is connected or connectable with a pouch (24), **characterized in that** the through opening (20) in the base plate (18) is formed by blanking out or punching out, wherein, in particular size and/or shape of the stoma (10) are determined, and **in that** the through opening (20) is prepared in size and/or shape so as to fit to the stoma (10).

15. Use of an ostomy care system (28) in accordance with any one of claims 1 to 13 for carrying out a method according to claim 14.
